# EUROPEAN PATENT SPECIFICATION

(11) **EP 0 293 228 B1**
(45) Date of publication and mention of the grant of the patent: **20.10.1993**
(21) Application number: 88304836.5
(22) Date of filing: 27.05.1988
(51) Int. Cl.: F16M 11/12, G02B 7/00

(54) **Stand mechanism for a medical optical equipment**
Stativ für ein medizinisches Optikgerät
Support d'équipement optique médical

(30) Priority: 29.05.1987 JP 134500/87
(43) Date of publication of application: 30.11.1988
(73) Proprietor: Mitaka Kohki Co., Limited, Mitaka-shi Tokyo (JP)
(72) Inventor: Nakamura, Katushige, Hachioji-shi Tokyo (JP)
(74) Representative: Gold, Tibor Z.

(56) References cited:
- EP-A- 0 023 003
- EP-A- 0 202 399
- DE-A- 1 572 597
- FR-A- 2 002 214
- US-A- 3 945 597

## Description

### Background of the Invention

The present invention relates to a stand mechanism for supporting medical optical equipment.

An encephalic surgical operation and a cardiac surgical operation are implemented by observing the diseased region with medical optical equipment, e.g. a surgical microscope, and such very delicate operations impose a high strain on the nerves and, in most cases, require a long time, which undesirably causes both the operators and the patient physical and spiritual fatigue.

Medical optical equipment such as a surgical microscope plays a very significant role in advanced surgical operations, and the accessibility of the medical optical equipment has a direct effect on reducing time necessary for a surgical operation. The accessibility of the medical optical equipment, namely, the possibility of positioning the medical optical equipment accurately and quickly at an objective position corresponding to the diseased region, the possibility of moving the medical optical equipment away from the region of the operation to and fixedly positioning the same at an optional standby position, and the stability of the medical optical equipment at a fixed position, are all dependent mostly on the performance of a stand mechanism supporting the medical optical equipment.

Various stand mechanisms for supporting a medical optical equipment including such as disclosed in Japanese Patent Provisional Publicaon (Kokai) No.56-32110 have been proposed. However, these known stand mechanisms are not sufficiently satisfactory in respect of accessibility, in practising medical operations.

A further stand mechanism is also disclosed in FR-A-2 002 214, which has a rotary shaft and a parallelogram linkage for supporting optical equipment with its focus held on a line forming an extension of the longitudinal axis of the shaft. The movement of the shaft and linkage is motor controlled, and counterweights provide a partial counterbalancing effect, whereby free movement and stable positioning of the equipment is restricted.

### Brief Summary of the Invention

The present invention has been made in view of the above-mentioned disadvantages of the prior art and it is therefore an object of the present invention to provide a stand mechanism for supporting medical optical equipment capable of properly functioning in practical medical operations and capable of enabling the medical optical equipment to demonstrate all its functions thereof.

The present invention provides a stand mechanism according to claim 1.

Various other features and advantages of the invention will be more apparent by referring to the following description and the accompanying drawings.

### Brief Description of the Drawings

Figure 1 is a general perspective view of a stand mechanism for medical optical equipment, embodying the present invention;
Figure 2 is a schematic side elevation showing the weight balancing system of the stand mechanism of Figure 1;
Figure 3 is a schematic end elevation as viewed in the direction of an arrow III in Figure 2;
Figure 4 is a diagrammatic illustration for assisting in explaining the operation of a parallel linkage in adjusting the observation angle of medical optical equipment in a plane including the parallel linkage;
Figure 5 is a fragmentary perspective view of a mechanism for swinging a counterweight according to the pivotal motion of a swivel plate in a horizontal plane;
Figure 6 is a schematic plan view for assisting in explaining the motion of the stand mechanism of Fig. 1 in moving the medical optical equipment in longitudinal directions;
Figure 7 is a schematic plan view for assisting in explaining the motion of the stand mechanism of Fig. 1 in moving the medical optical equipment in lateral directions;
Figure 8 is a side elevation of the medical optical equipment disposed in a vertical position;
Figures 9(a) to 9(c) are side elevations showing the process of changing the position of the medical optical equipment from a vertical position to a 45°-inclined position; and
Figures 10(a) to 10(c) are side elevations showing processes of changing the position of the medical optical equipment from a vertical position to a horizontal position.

### Description of the Preferred Embodiments

A preferred embodiment of a stand mechanism for supporting a medical optical equipment according to the present invention will be described hereinafter with reference to the accompanying drawings.

First the construction of the stand mechanism is described, and then the operation of the stand mechanism will be described.

A central shaft 21 is supported rotatably in bearings in an inclined holding unit 22. The front end projecting in a direction indicated by an arrow A of the central shaft 21 is machined to form a plate-shaped portion. Parallel links 23 and 24 of a parallel linkage 25 are joined pivotally at the respective base ends 26 thereof to the plate-shaped portion of the central shaft 21.

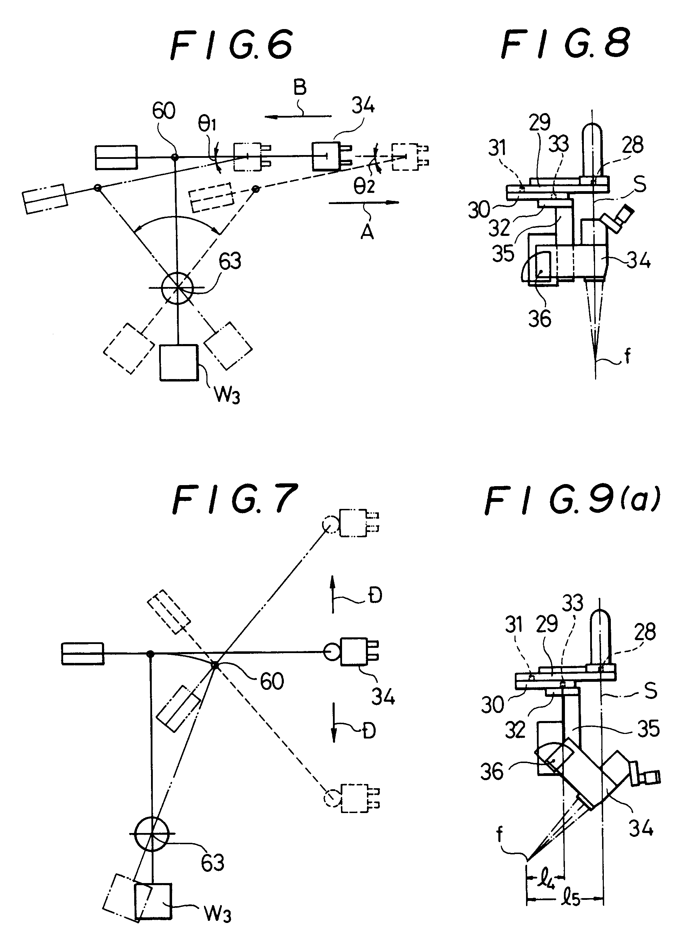

A swivel member 29 serving as a support is joined pivotally to the extremity 27 of the parallel linkage 25 for turning motion about an axis 28 of rotation in a horizontal plane. A first swivel plate 30 is joined pivotally to the free end of the swivel member 29 by way of a pivot shaft 31 for facilitating turning motion about the pivot shaft 31 in a horizontal plane, see Fig. 8. A second swivel plate 32 is joined pivotally to the free end of the first swivel plate 30 with a pivot shaft 33 for turning motion in a horizontal plane. A suspending arm 35 is supported by the second swivel plate 32, and a surgical microscope 34 (medical optical equipment) is supported for pivotal movement on the suspending arm 35 directly below the second swivel plate 32 so as to be turnable about an axis 36 of rotation between a vertical position, a 45°-inclined position where the surgical microscope 34 is inclined at an angle of 45° and a horizontal position where the surgical microscope is inclined at an angle of 90° from the vertical. The focus f of the surgical microscope 34 stays always on a vertical line S passing the axis 28 of rotation regardless of the position of the surgical microscope, which will be described in detail below. Moreover, since the focus f of the surgical microscope 34 is positioned on the extension L of a straight line connecting the base ends 26 of the parallel linkage 25 aligned with the extension of the central axis of central shaft 21, the position of the focus f remains unchanged when the parallel linkage is transformed. A handle 37 is provided for moving (swinging) the surgical microscope 34. Fixtures 38 are provided respectively at the base end and front end of the swivel plate 29 to change the position of the handle 37 according to the position of the operator.

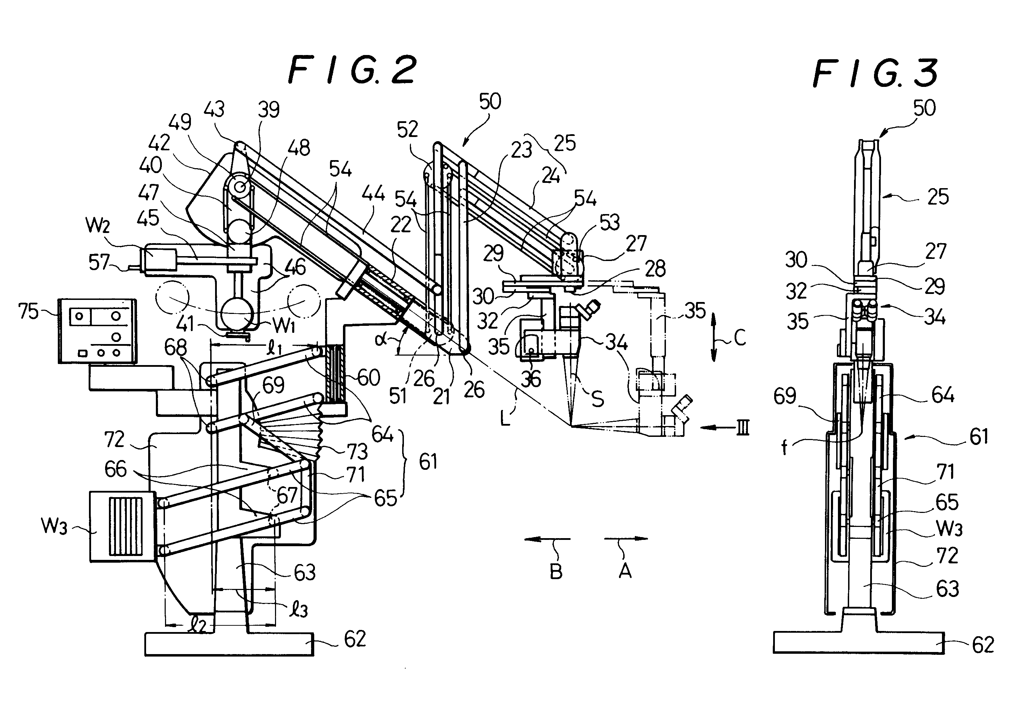

A first pendulous counterweight W1 is suspended by a swing bar 40 from the rear end projecting in a direction indicated by an arrow B of the central shaft 21 (Fig 2). so as to be able to swing on a pivot shaft 39. An adjusting handle 41 is associated with the first counterweight W1 to adjust the vertical position of the first counterweight W1. The base portion of the swing bar 40 is covered with a cover 42. The first counterweight W1 and a second counterweight W2 are covered with another cover 46.

The upper end 43 of the swing bar 40 and the parallel linkage 25 are interconnected by a connecting bar 44 to interlock the parallel linkage 25 and the first counterweight W1. Thus, the first counterweight W1 counterbalances the inclusive weight of the surgical microscope 34 and the parallel linkage 25, and the rotary moment of the parallel linkage 25 with respect to the central axis of the central shaft 21. Since the central shaft 21 is inclined at an angle α to a horizontal line, the first counterweight W1 and the associated parts are excluded from an operating zone, whereby a sufficiently wide and free operating zone can be secured for the operator.

The second counterweight W2 is connected by another swing bar 45 to the middle of the swing bar 40. The second counterweight W2 is able to swing in a vertical plane to the front and rear together with first counterweight W1 and is able to be swung in a horizontal plane by a mechanism which will be described below. That is, the base end of the swing bar 45 for the second counterweight W2 is fixed to a pivot shaft 47 which is coaxial with the swing bar 40, and the second counterweight W2 moves along a semicircular path behind with respect to the pivot shaft 47 (Fig. 5).

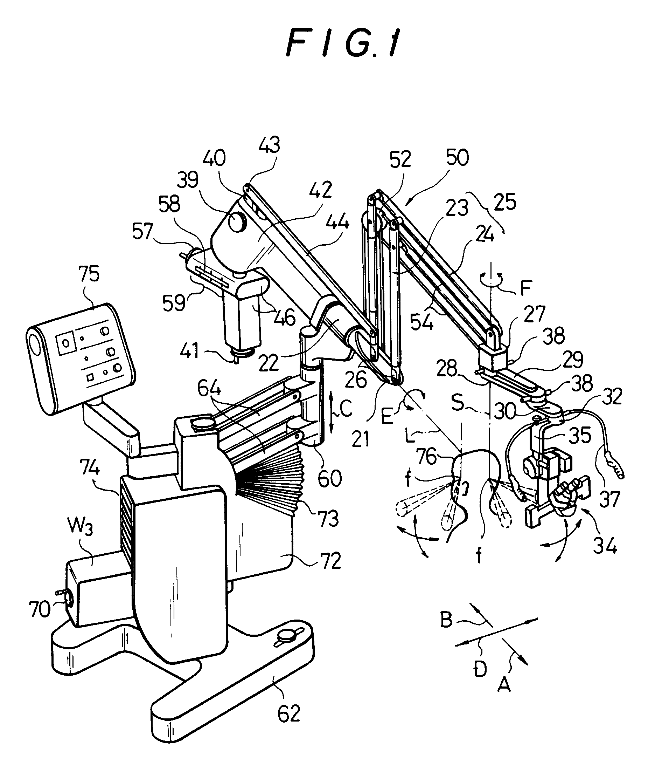

The pivot shaft 47 is interlocked with a rotary disk 49 provided near the rear end of the central shaft 21 through a pair of bevel gears 48 respectively having axes of rotation perpendicular to each other. Disks 51, 52 and 53, which are similar to the disk 49, are provided respectively near the base ends 26 of the parallel linkage 25, an articulated portion 50 of the parallel linkage 25 and the extremity 27 of the parallel linkage 25. One of the bevel gears 48 and the disks 49, 51, 52 and 53 are interlocked by pairs of link bars 54 to transmit torque therethrough. The disk 53 provided near the extremity 27 of the parallel linkage 25 is interlocked with the swivel plate 29 by means of a pair of bevel gears 55. One of the bevel gears 55 is fixed to the disk 53 and the other bevel gear 55 is fixed to a pivot shaft 56 fixed to the swivel plate 29 and engages the bevel gear 55. Accordingly, when the swivel plate 29 is pivoted in a horizontal plane, the turning motion of the swivel plate 29 is transmitted through the pivot shaft 56, the bevel gears 55, the disk 53, the disk 52, the disk 51, the disk 49, the bevel gears 48 and the pivot shaft 47, in that order, to the swing bar 45 supporting the second counterweight W2 to turn the swing bar 45 in a horizontal plane in a direction which is the same as that of turning motion of the swivel plate 29, so that the swivel plate 29 and the swing bar 45 supporting the second counterweight W2 extend respectively in opposite directions. Thus, the rotary moment with respect to the central shaft 21 produced by the pivotal motion of the swivel plate 29 is counterbalanced. The position of the second counterweight W2 on the swing bar 45 can also be adjusted according to the inclination (0°, 45° or 90°) of the surgical microscope 34 by turning an adjusting handle 57 shown in Figs. 1 and 2. A slit 58 is formed in a portion of the cover 46 covering the second counterweight W2. Three notches 59 are formed in the slit 58 at positions corresponding respectively to the three inclinations of the surgical microscope 34. The position of the second counterweight W2 on the swing bar 45 can easily and accurately be adjusted according to the inclination of the surgical microscope 34 by turning the adjusting handle 57 to bring a countermark on the second counterweight W2 into alignment with one of the notches 59 corresponding to the inclination of the surgical microscope 34.

The holding unit 22 pivotally holding the central shaft 21 has a lower cylindrical part 60 rotatably supported in bearings. The cylindrical part 60 is joined to the links of a multiple parallel linkage 61, (Fig. 2). Thus, the multiple parallel linkage 61 supports the holding unit 22 and the cylindrical part 60. The multiple parallel linkage 61 supports the inclusive weight of the central shaft 21 and all the components supported on the central shaft 21 for vertical movement. The multiple parallel linkage 61 is supported pivotally on a swivel stand shaft 63 set upright for swivel motion on a base 62. The multiple parallel linkage 61 has two pairs of upper parallel links 64 and two pairs of lower parallel links 65 disposed in parallel one over the other. The lower parallel links 65 are supported pivotally respectively on pivot shafts 67 attached respectively to the extremities of projections 66 projecting from the stand shaft 63. The upper parallel links 64 are supported pivotally above the lower parallel links 65 respectively on pivot shafts 68 provided near the upper end of the stand shaft 63. The upper parallel links 64 and the lower parallel links 65 are interlocked with each other by connecting links 69. A third counterweight W3 for counterbalancing the inclusive weight of the central shaft 21 and the components supported on the central shaft 21 (hereinafter referred to as "the upper structure") is joined to the rear ends of the lower parallel links 65. The position of the third counterweight W3 can be adjusted by an adjusting handle 70 shown in Fig. 1. If a single parallel linkage is used instead of the upper parallel linkage essentially consisting of the upper parallel links 64 having an effective length ℓ₁ (i.e. the distance between the holding unit 22 and the pivot shafts 68) and the lower parallel linkage essentially consisting of the lower parallel links 65 having an effective length ℓ₂ (i.e. the distance between the pivot shafts 67 and the third counterweight W3) so as to support the upper structure, the effective length of the links of the single parallel linkage is the sum of the effective lengths ℓ₁ and ℓ₂. However, the use of the upper parallel links 64 and the lower parallel links 65 permits the size of the stand mechanism to be determined by the horizontal distance ℓ₃ between the pivot shafts 67 and the pivot shafts 68, which enables the stand mechanism to have a compact construction. Furthermore, since the rearward projection of the third counterweight W3 is small, the third counterweight W3 will not interfere with peripheral equipments, which is advantageous from the viewpoint of securing safety. Connecting links 71 are provided to reinforce the lower parallel links 65. The stand shaft 63 and the lower parallel links 65 are covered with a cover 72. Openings in the cover 72 for the vertical movement of the upper parallel links 64 and the third counterweight W3 are covered with bellows covers 73 and 74, respectively. Indicated at 75 is a control panel. The stand mechanism has electromagnetic clutches, not shown, provided respectively at the articulated portions and operated selectively by means of a foot switch or the like to lock or free the components selectively.

The operation of the stand mechanism will be described hereinafter.

### Longitudinal Motion (Directions A and B)

As shown in Fig. 6, when the surgical microscope is moved in a longitudinal direction, the stand shaft 63 and the cylindrical part 60 are pivoted accordingly. An angle ϑ₁ or ϑ₂ of deviation of the surgical microscope 34 with respect to the parallel linkage 25 due to the movement of the surgical microscope 34 in the direction of the arrow B or A is compensated automatically with the turning of the swivel plate 29 holding the surgical microscope 34 in one of the directions indicated by a double-headed arrow F, and hence the surgical microscope 34 can be translated in longitudinal directions without varying the observation angle.

### Vertical Motion (Directions C)

When the surgical microscope 34 is moved in a vertical direction, the multiple parallel linkage 61 is transformed and the upper structure is moved in the same direction. Since the moment of the upper structure is counterbalanced by that of the third counterweight W3, the upper structure can be moved in an effortless manner in vertical directions.

### Lateral Motion (Directions D)

As shown in Fig. 7, when the surgical microscope 34 is moved in a lateral direction, the stand shaft 63 and the cylindrical part 60 pivot accordingly. Since the angle of deviation of the surgical microscope 34 with respect to the parallel linkage 25 attributable to the longitudinal movement of the surgical microscope 34 is compensated automatically with the pivotal motion of the swivel plate 29 holding the surgical microscope 34 in one of the directions of the double-headed arrow F, the surgical microscope 34 can be translated in lateral directions without varying the observation angle.

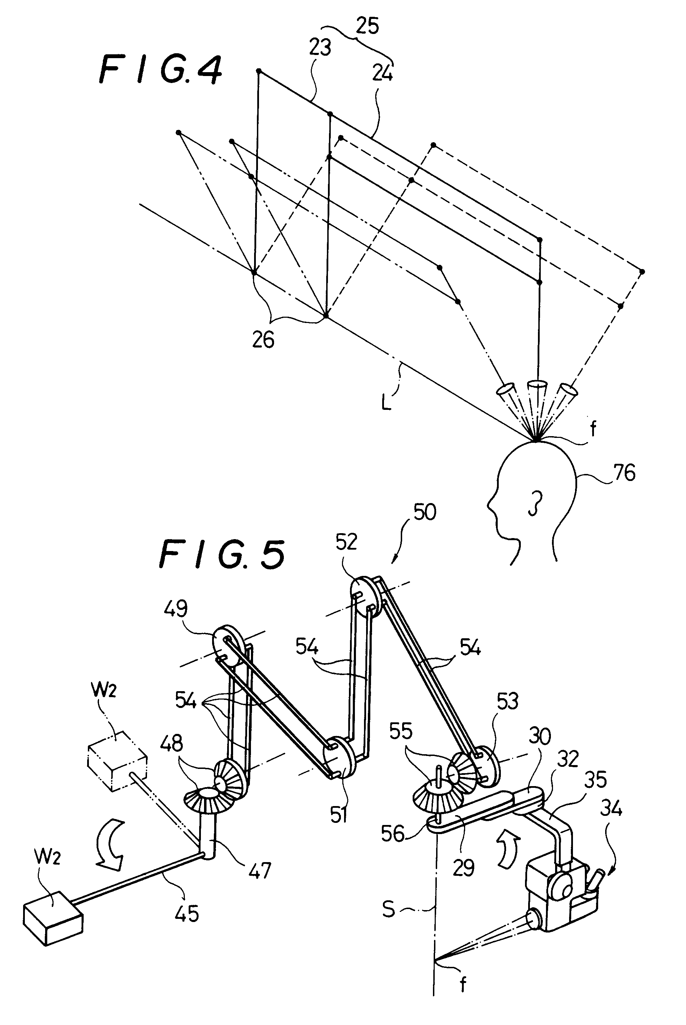

### Adjustment of Inclination of Surgical Microscope (Fig. 4)

Since the focus f of the surgical microscope 34 is located on the extension L of the straight line connecting the base ends 26 of the parallel linkage 25, namely, on the extension of the central axis of the central shaft 21, the focus f remains fixed at its original position when the parallel linkage 25 is transformed. Accordingly, the observation angle can optionally be adjusted in a plane including the parallel linkage 25. When the parallel linkage 25 is transformed, both the first counterweight W1 and the second counterweight W2 are caused to swing. Therefore, the inclusive weight of the surgical microscope 34 and the parallel linkage 25 is counterbalanced by the first counterweight W1 and the second counterweight W2 automatically to maintain balanced stability. The position of the first counterweight W1 on the swing bar 45 must be adjusted by means of the adjusting handle 41 according to the weight of any accessories, such as a microscope for an assistant and a video camera, that may additionally be mounted on the surgical microscope 34.

### Turning Motion of Central Shaft (Directions E)

When the surgical microscope 34 is pivoted about the central axis of the central shaft 21, the parallel linkage 25 is likewise pivoted about the central axis of the central shaft 21 in the same direction, and hence the first counterweight W1 and the second counterweight W2 are swung in the same direction, together with the swing bar 40. Accordingly, balanced stability is maintained and hence the surgical microscope 34 is positioned at an optional stationary position.

### Horizontal Pivoting of Swivel Plate (Directions F)

Since the swivel plate 29 is capable of swivel motion in a horizontal plane, the surgical microscope 34 can be turned around the head 76 of the patient in directions of the double-headed arrow F. When the swivel plate 29 deviates from a position which represents an extension of the parallel linkage 25, for example, when the swivel plate 29 is turned to a position where the swivel plate 29 extends perpendicularly to the extension of the parallel linkage 25, the swing bar 45 holding the second counterweight W2 is turned through by means of the disks 49, 51, 52 and 53, the link bars 54 and the bevel gears 48 and 55 to a position where the swing bar 45 extends in the opposite direction with respect to the swivel plate 29. Accordingly, a balanced stability is maintained and hence the surgical microscope 34 will not turn automatically about the central axis of the central shaft 21 further in directions of the double-headed arrow E.

Furthermore, since the inclination of the swing bar 40 supporting the second counterweight W2 is always the same as that of the surgical microscope 34 owing to the functions of the parallel linkage 25 and the connecting arm 44, the second counterweight W2 functions perfectly in maintaining balanced stability regardless of the position of the swivel plate 29, and hence balanced stability with respect to the pivotal directions of the central shaft 21 is never destroyed.

Still further, since the second counterweight W2 swings behind about the pivot shaft 47 without varying the center of gravity of the upper structure, the counterbalancing function of the third counterweight W3 is constantly maintained.

### Changing The Observation Angle by Tilting The Surgical Microscope

In some cases, the observation angle of the surgical microscope 34 needs to be changed through a large angle depending on the position of the operative field. For example, an encephalic surgical operation includes the operation of the parietal region of the head 76 and the operation of the temporal region of the head 76. The stand mechanism according to the preferred embodiment of this invention is provided with the first swivel plate 30 and the second swivel plate 32 to enable a change of the observation angle in a wide angular range.

### Vertical Position:

Both the first swivel plate 30 and the second swivel plate 32 are pivoted towards folded positions below the swivel plate 29 so as to extend toward the axis 28 of rotation of the swivel plate 29, as shown in Fig. 8.

### 45°-Inclined Position:

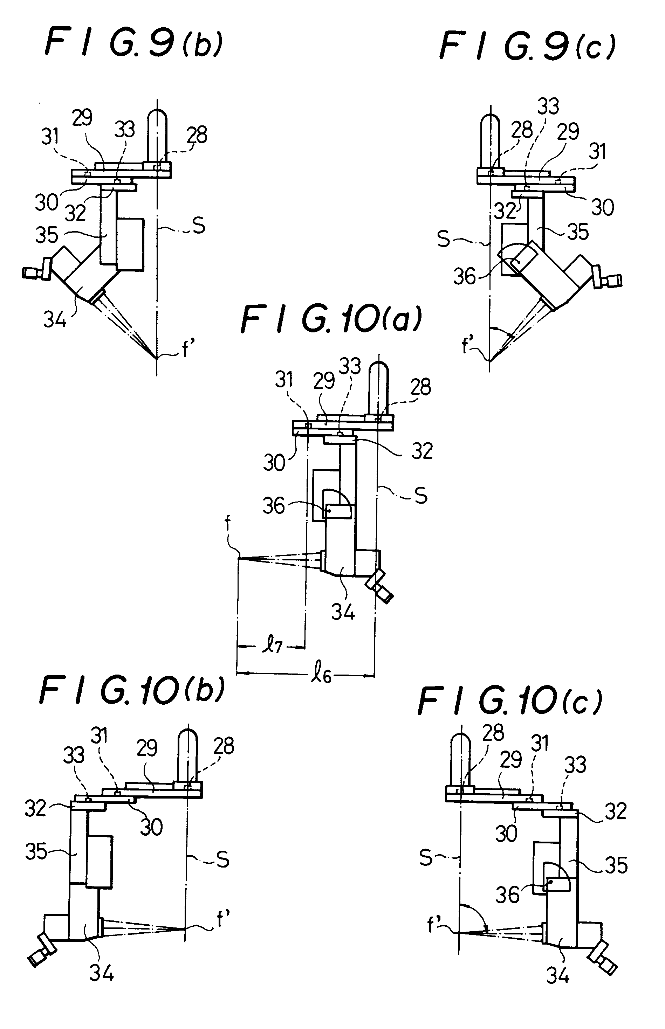

First, the surgical microscope 34 is tilted from the vertical position through an angle of 45° about the axis 36 of rotation (Fig. 9(a)). In this state, the focus f' is located on a vertical line at a distance ℓ₅ from the vertical line S on which the focus f is located when the surgical microscope 34 is in a vertical position and at a distance ℓ₄ from the central axis of the pivot shaft 33. The distance ℓ₅ is exactly twice the distance ℓ₄. Accordingly, when the second swivel plate 32 is turned through an angle of 180° on the pivot shaft 33, the focus f' is located on the vertical line S (Fig. 9(b)). Then, when the swivel plate 29 is turned through an angle of 180° about the axis 28 of rotation, the focus f' remains on the vertical line S and the operator is able to observe the operative field through the surgical microscope 34 from the same position, notwithstanding the fact that the surgical microscope 34 is tilted at an inclination of 45° (Fig. 9(c)). Accordingly, the focus f' can be brought to the original position of the focus f by slightly moving the surgical microscope 34 in a vertical direction by a lifting mechanism, not shown, incorporated into the surgical microscope 34.

### 90°-Inclined (Horizontal) Position:

The surgical microscope 34, which is disposed in a vertical position, is pivoted through an angle of 90° about the axis 36 of rotation (Fig. 10(a)). In this state, the focus f' is located on a vertical line at a distance ℓ₆ from the vertical line S and at a distance ℓ₇ from the central axis of the pivot shaft 31. The distance ℓ₆ is exactly twice the distance ℓ₇. Accordingly, the focus f' can be located on the vertical line S by swinging the first swivel plate 30 through an angle of 180° on the pivot shaft 31 (Fig. 10(b)). Then, when the swivel plate 29 is swung through an angle of 180° about the axis 28 of rotation, similarly to the case of 45°-inclined position, the focus f' remains on the vertical line S and the operator is able to observe the operative field through the surgical microscope 34 from the same position, notwithstanding the fact that the surgical microscope 34 is now horizontal, having swung through 90° (Fig. 10(c)).

Although the multiple parallel linkage 61 of the stand mechanism in this embodiment is a two-unit parallel linkage, the multiple parallel linkage may be a three-unit parallel linkage or a multiple parallel linkage having more than three units. Furthermore, although the invention has been described as applied to supporting a surgical microscope as an example of a medical optical equipment, the present invention is applicable to supporting various other medical equipments, e.g. medical laser equipment. Still further, although the stand mechanism in this embodiment is a floor-type stand mechanism, the present invention is applicable also to a suspension stand mechanism for the same function.

The stand mechanism for a medical optical equipment according to the present invention provides the following effects.
(a) Since the focus of the medical optical equipment remains fixed at a fixed position when the parallel linkage is transformed, the medical optical equipment does not need to be focused even when the parallel linkage is operated, and thereby operating times may be appreciably reduced. Furthermore, since the observation angle may be changed while the focus is maintained on a fixed point, the diseased region may be observed from an optimal observation angle, and hence the operation may be carried out more precisely and more rapidly.
(b) Since the inclusive weight of the optical equipment and the parallel linkage, and the rotary moment of the parallel linkage with respect to the central axis of the central shaft are counterbalanced by the counterweights, the stand mechanism is formed as a simple construction and is able to operate smoothly in a satisfactory balanced condition of the weights without generating noises and squeak. The employment of the rotary central shaft eliminates the need of a counterweight for balancing the weight of the parallel linkage with respect to the direction of pivoting and simplifies the construction of the stand mechanism.
(c) Since the rotary moment with respect to the axis of the central shaft resulting from the pivoting motion of the supporting unit is counterbalanced by the counterweight, balanced stability about the central shaft is maintained when the supporting unit supporting heavy medical optical equipment is turned in a horizontal plane and the medical optical equipment can be stopped at any desired position.
(d) Since the supporing unit is supported for vertical movement on the extremity of the uppermost parallel linkage of the multiple parallel linkage, and the counterweight for counterbalancing the weight of the supporting unit is provided on the rear end of the lowermost parallel linkage of the multiple parallel linkage, the upper structure may easily be moved vertically. Moreover, the stand mechanism has a pleasing appearance and, since the counterweight is not projected backwardly the counterweight will not interfere with the operator and any peripheral equipments, which is advantageous from the viewpoint of safety.
(e) Since the focus of the optical equipment in the 45°-inclined position or in the horizontal (90°-inclined) position can be located at a position where the focus is located when the optical equipment is in the vertical position by turning the first swivel plate or the second swivel plate through an angle of 180° in a horizontal plane, the medical optical equipment can be used at an optimum observation angle. Moreover, since the focus moves along the same vertical line, the medical optical equipment may be focused rapidly, thereby appreciably remarkably reducing the time required for a surgical operation.

## Claims

1. A stand mechanism for supporting medical optical equipment (34) comprising parallel linkage means (25) in the form of cinematically interdependent parallelogram links (23, 24) for supporting said optical equipment (34);
a rotary shaft (21) rotatable about a longitudinal axis (E) thereof and having said parallel linkage means (25) connected thereto at predetermined locations along said longitudinal axis (E) such that the focus (f) of said optical equipment (34) is always focused upon a line (L) which is an extension of said longitudinal axis (E), a first one of said links (23) of said parallel linkage means (25) extending substantially radially outwardly from said longitudinal axis, and a second one of said links (24) extending substantially parallel to said longitudinal axis for supporting said optical equipment (34);
counterweight means (W1, W2) mounted on said rotary shaft (21) and pivotable in a plane defined by said parallel linkage means (25) ; and
said parallel linkage means (25) and said counterweight means (W1, W2) being interlocked by first interlocking means (40, 43, 44) characterised in that shaft (21) is freely rotatable and said parallel linkage means (25) is freely translatable, in that said parallel linkage means (25) and said counterweight means (W1, W2) are interlocked in such a way that the total inclusive weight of the optical equipment (34) together with that of the parallel linkage means (25) and the torque of the parallel linkage means (25) and of optical equipment (34) with respect to said shaft (21) are exactly counterbalanced by said counterweight means (W1, W2), and in that a swivel member (29) is pivotably connected to said second link (24) for rotation about a first axis of rotation (28) parallel to said first link (23) and at least a part (W2) of said counterweight means (W1, W2) is pivotable about an axis (47) parallel to said first axis of rotation (28) said swivel member (29) and said part (W2) of said counterweight being interlocked by second interlocking means (45, 48, 49, 50-55) such that the rotary moment caused by said swivel member (29), and any equipment supported thereby, with respect to the longitudinal axis (E) of the rotary shaft (21) is counterbalanced by said part (W2).

2. A stand mechanism according to claim 1, characterised by a first swivel plate (30) connected to said swivel member (29) so as to be rotatable about a second axis (31) of rotation parallel to said first axis of rotation (28); a second swivel plate (32) connected to said first swivel plate (30) so as to be rotatable about a third axis (33) of rotation which is parallel to said first and second axes of rotation, and means (35) for pivotably mounting said optical equipment (34) on said second swivel plate (32) so as to be capable of disposing said optical equipment (34) in vertical, inclined and horizontal modes about a horizontal axis while maintaining the latter (34) focused on said line (L).

## Patentansprüche

1. Ein Stativ für ein medizinisches Optikgerät (34) bestehend aus: Parallelkopplungsmittel (25) in Form von kinematisch voneinander abhängigen Parallelogrammgliedern (23, 24) zum Tragen des Optikgerätes (34);
einem Drehschaft (21), der um seine Längsachse (E) drehbar ist und besagte Parallelkopplungsmittel (25) aufweist, die an bestimmten Stellen entlang der Längsachse (E) mit ihm verbunden sind derart, daß der Brennpunkt (f) des Optikgerätes (34) immer auf eine Linie (L) eingestellt ist, die eine Verlängerung der Längsachse (E) ist, wobei ein erstes der Parallelogrammglieder (23) der besagten Parallelkopplungsmittel (25) sich im wesentlichen von der Längsachse radial nach außen erstreckt, und ein zweites der Parallelogrammglieder (24) sich im wesentlichen parallel zu der Längsachse erstreckt, um das Optikgerät (34) zu tragen;
Gegengewichtmitteln (W1, W2), die an dem Drehschaft (21) befestigt und in einer durch die besagten Parallelkopplungsmittel (25) definierten Ebene schwenkbar sind;
wobei die Parallelkopplungsmittel (25) und die Gegengewichtsmittel (W1, W2) durch erste Verriegelungsmittel (40, 43, 44) verriegelt sind,
dadurch gekennzeichnet,
daß der Drehschaft (21) frei drehbar und die Parallelkopplungsmittel (25) frei verstellbar sind, daß die Parallelkopplungsmittel (25) und die Gegengewichtsmittel (W1, W2) so verriegelt sind, daß das alles umfassende Gesamtgewicht des Optikgerätes (34) zusammen mit demjenigen der Parallelkopplungsmittel (25) und die Drehkraft der Parallelkopplungsmittel (25) und des Optikgerätes (34) bezüglich des Drehschaftes (21) genau durch die Gegengewichtsmittel (W1, W2) ausgeglichen sind, und daß ein Drehelement (29) schwenkbar mit dem zweiten Parallelogrammglied (24) verbunden ist für Drehungen um eine erste Drehachse (28), die parallel zu dem ersten Parallelogrammglied (23) verläuft, und daß zumindest ein Teil (W2) der Gegengewichtsmittel (W1, W2) schwenkbar um eine Achse (47) ist, die parallel zu der ersten Drehachse (28) ist, wobei das Drehelement (29) und besagter Teil (W2) der Gegengewichtsmittel durch zweite Verriegelungsmittel (45, 48, 49, 50 - 55) derart verriegelt sind, daß das durch das Drehelement (29) und eventuelles hiervon getragenes Zubehör verursachte Drehmoment in Bezug auf die Längsachse (E) des Drehschaftes (21) durch besagten Gegengewichtsteil (W2) ausgeglichen ist.

2. Stativ nach Anspruch 1, gekennzeichnet durch eine erste Drehplatte (30), die mit dem Drehelement (29) so verbunden ist, daß sie um eine zweite Drehachse (31) drehbar ist, welche parallel zu der ersten Drehachse (28) ist; eine zweite Drehplatte (32), die derart mit der ersten Drehplatte (30) verbunden ist, daß sie drehbar um eine dritte Drehachse (33) ist, die parallel zur ersten und zur zweiten Drehachse ist, und Mittel (35), um das Optikgerät (34) auf der zweiten Drehplatte (32) schwenkbar anzuordnen, so daß es möglich ist, das Optikgerät (34) vertikal, schräg und horizontal um eine Horizontalachse zu bewegen, wobei das Optikgerät (34) auf besagter Linie (L) fokussiert bleibt.

## Revendications

1. Mécanisme support adapté à supporter un équipement optique médical (34), comprenant des moyens de liaison parallèles (25) se présentant sous la forme de bielles en parallélogramme (23, 24) cinématiquement interdépendantes pour supporter ledit équipement optique (34);
- un arbre rotatif (21) adapté à tourner autour de son axe londitudinal (E) et auquel lesdits moyens de liaison parallèles (25) sont connectés en des emplacements prédéterminés le long dudit axe longitudinal (E) de telle sorte que le foyer (f) dudit équipement optique (34) est toujours au point sur une liqne (L) qui est un prolongement dudit axe longitudinal (E), une première desdites bielles (23) desdits moyens de liaisons parallèles (25) s'étendant sensiblement radialement vers l'extérieur depuis ledit axe longitudinal et une seconde desdites bielles (24) s'étendant sensiblement parallèlement audit axe longitudinal pour supporter ledit équipement optique (34);
- des moyens formant contrepoids (W1, W2) montés sur ledit arbre rotatif (21) et pivotant dans un plan défini par lesdits moyens de liaison parallèles (25)
- lesdits moyens de liaison parallèles (25) et lesdits moyens formant contrepoids (W1, W2) étant rendus interdépendants par des premiers moyens de mise en dépendance (40̸, 43, 44),
caractérisé en ce que ledit arbre (21) tourne librement et en ce que les moyens de liaisons parallèles (25) peuvent être déplacés librement en translation, en ce que lesdits moyens de liaisons parallèles (25) et lesdits moyens formant contrepoids (W1, W2) sont rendus interdépendants de telle manière que le poids total de l'équipement optique (34) avec celui des moyens de liaison parallèles (25) et le moment des moyens de liaison parallèles (25) et de l'èquipement optique (34) par rapport audit arbre (21) sont exactement contre-balancés par lesdits moyens formant contrepoids (W1, W2) et en ce qu'un élément orientable (29) est connecté de façon pivotante sur ladite seconde bielle (24) pour pouvoir tourner autour d'un premier axe de rotation (28) parallèle à ladite première bielle (23) et au moins une partie (W2) desdits moyens formant contrepoids (W1, W2) peut pivoter autour d'un axe (47) parallèle audit premier axe de rotation (28), ledit élément orientable (29) et ladite partie (W2) dudit contrepoids étant rendus interdépendants par des seconds moyens de mise en dépendance (45, 48, 49, 50̸-56) de telle sorte que le mouvement de rotation causé par ledit élément orientable (29) et tout équipement supporté par celui-ci, par rapport à l'axe longitudinal (E) de l'arbre rotatif (21), est contrebalancé par ladite partie (W2).

2. Mécanisme support selon la revendication 1, caractérisé par une première plaque orientable (30̸) connectée audit élément orientable (29) de façon à pouvoir tourner autour d'un second axe (31) de rotation parallèle audit premier axe de rotation (28), une seconde plaque orientable (32) connectée à ladite première plaque (30̸) de façon à pouvoir tourner autour d'un troisième axe (33) de rotation qui est parallèle auxdits premier et second axes de rotation, et des moyens (35) pour le montage pivotant dudit équipement optique (34) sur ladite seconde plaque orientable (32) de façon à pouvoir disposer ledit équipement (34) en mode vertical, incliné ou horizontal autour d'un axe horizontal tout en maintenant ledit équipement (34) au point sur ladite ligne (L).
